(19) Europäisches Patentamt
European Patent Office
Office européen des brevets

(11) **EP 4 666 943 A1**

(12) **EUROPEAN PATENT APPLICATION**
published in accordance with Art. 153(4) EPC

(43) Date of publication:
**24.12.2025 Bulletin 2025/52**

(21) Application number: **24756858.7**

(22) Date of filing: **13.02.2024**

(51) International Patent Classification (IPC):
**A61B 5/11** (2006.01)     **G06T 7/00** (2017.01)
**G06T 7/20** (2017.01)     **G06V 40/20** (2022.01)

(52) Cooperative Patent Classification (CPC):
**A61B 5/11; G06T 7/00; G06T 7/20; G06V 40/20**

(86) International application number:
**PCT/JP2024/004768**

(87) International publication number:
**WO 2024/172013 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.02.2023 JP 2023019703**

(71) Applicants:
• **University Public Corporation Osaka**
**Osaka-shi, Osaka 5360025 (JP)**
• **Iida Sangyo Co., Ltd.**
**Musashino-shi, Tokyo 180-0022 (JP)**

(72) Inventors:
• **SUZUKI, Yuta**
**Osaka-shi, Osaka 558-8585 (JP)**
• **OKAZAKI, Kazunobu**
**Osaka-shi, Osaka 558-8585 (JP)**
• **SUZUKI, Takashi**
**Osaka-shi, Osaka 558-8585 (JP)**
• **MORI, Kazuhiko**
**Musashino-shi, Tokyo 180-0022 (JP)**
• **MATSUMOTO, Koichi**
**Musashino-shi, Tokyo 180-0022 (JP)**

(74) Representative: **Mewburn Ellis LLP**
**Aurora Building**
**Counterslip**
**Bristol BS1 6BX (GB)**

(54) **COORDINATE TRANSFORMATION METHOD, COORDINATE TRANSFORMATION DEVICE, MOTION ANALYSIS METHOD, MOTION ANALYSIS DEVICE, AND COMPUTER PROGRAM**

(57)     The object of the present invention is to provide a coordinate transformation procedure that allows for detailed analysis of motion such as walking by using motion information collected from one position. The present invention is a coordinate transformation method characterized by carrying out a procedure to: acquire three-dimensional skeletal information of a subject in motion who is described under a first coordinate system comprising a x' direction, a y' direction, and a z' direction, which are mutually orthogonal; use the skeletal information to calculate a Y direction being the traveling direction of the subject, a Z direction along the torso of the subject, and a X direction orthogonal to the Y direction and the Z direction; and transform the first coordinate system into a second coordinate system comprising the X direction, the Y direction, and the Z direction.

FIG. 6

**Description**

TECHNICAL FIELD

The present invention relates to a coordinate transformation method, a coordinate transformation device, a motion analysis method, a motion analysis device, and a computer program that can be suitably used for analyzing movements such as walking.

BACKGROUND ART

Markerless motion capture systems enable simple motion analysis, and are expected to be used in a variety of fields in the future. On the other hand, in order to perform detailed motion analysis using a markerless motion capture system, measurement accuracy and definition of the coordinate system become issues. In particular, in a movement of a subject such as walking, in which the subject moves in a certain direction, it is necessary to set an appropriate coordinate system (for example, a world coordinate system fixed to the ground) for analyzing the movement.

However, since many markerless motion capture systems use a coordinate system that is fixed to the system, the accuracy of the measured data becomes worse, and it is not possible to use the measured data directly for analyzing walking movements.

Alternatively, for example, Patent Document 1 (Japanese Patent No. 6381918) discloses a movement information processing device comprises: an acquisition unit that acquires pieces of movement information of a target person that are collected at various positions from the target person who is performing a predetermined movement, a position calculation unit that calculates association information for associating the respective pieces of movement information acquired by the acquisition unit, and a display control unit that controls an output unit to output the output information in which the respective pieces of operation information are associated on the basis of the association information calculated by the position calculation unit.

However, such an apparatus requires motion information collected from multiple positions, and therefore requires complex processing to align the acquired motion information.

PRIOR ART REFERENCE

MON-PATENT REFERENCE

Patent document 1: Japanese Patent No. 6381918

SUMMARY OF THE INVENTION

PROBLEM TO BE SOLVED BY THE INVENTION

An object of the present invention is to provide a coordinate transformation procedure that allows for detailed analysis of motion such as walking using motion information collected from a single position.

MEANS TO SOLVE THE PROBLEM

In order to solve the above-mentioned problems, a first aspect of the present invention provides coordinate transformation method characterized by carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction; and
transforming the first coordinate system into a second coordinate system comprising the X direction, the Y direction, and the Z direction, and

thereby acquiring coordinate-transformed skeletal information.

In the coordinate transformation method of the present invention, it is preferable that the Z direction is a direction from a pelvis to a chest of the subject.

In the coordinate transformation method of the present invention, it is preferable that, by using a matrix corresponding to the first coordinate system and a matrix corresponding to the second coordinate system, the first coordinate system is transformed into the second coordinate system.

A second aspect of the present invention provides a computer program for carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction; and
transforming the first coordinate system into a second coordinate system comprising the X direction, the Y direction, and the Z direction, and

thereby acquiring coordinate-transformed skeletal information.

A third aspect of the present invention provides a coordinate transformation device comprising:

an acquisition unit that acquires three-dimensional skeletal information of a subject in motion in a first coordinate system comprising a x' direction, a y' direction, and a z' direction, which are mutually orthogonal;
an axial direction calculation unit that, by using the skeletal information, calculates a Y direction being the traveling direction of the subject, a Z direction along the torso of the subject, and a X direction orthogonal to the Y direction and the Z direction; and
a coordinate transformation unit that transforms the first coordinate system into a second coordinate system comprising the X direction, the Y direction, and the Z direction.

A fourth aspect of the present invention provides a motion analysis method characterized by carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction;
transforming the first coordinate system into a second coordinate system comprising the X direction, the Y direction, and the Z direction; and
performing motion analysis using the coordinate-transformed skeletal information.

A fifth aspect of the present invention provides a computer program for carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction;
transforming the first coordinate system into a second coordinate system comprising the X direction, the Y direction, and the Z direction; and
performing motion analysis using the coordinate-transformed skeletal information.

A sixth aspect of the present invention provides a motion analysis device comprising:

an acquisition unit that acquires three-dimensional skeletal information of a subject in motion in a first coordinate system comprising a x' direction, a y' direction, and a z' direction, which are mutually orthogonal;
an axial direction calculation unit that, by using the skeletal information, calculates a Y direction being the traveling direction of the subject, a Z direction along the torso of the subject, and a X direction orthogonal to the Y direction and the Z direction;
a coordinate transformation unit that transforms the first coordinate system into a second coordinate system comprising the X direction, the Y direction, and the Z direction; and
an analysis unit that performs motion analysis using the coordinate-transformed skeletal information.

Here, each of the above methods is a computer-implemented method. Further, each of the above computer programs may be stored in a non-transitory computer-readable storage medium and executed by a processor.

EFFECT OF THE INVENTION

According to the present invention, since the motion information is written in an appropriately transformed coordinate system, motion such as walking can be analyzed in detail using motion information collected from the single position.

BRIEF EXPLANATION OF DRWAINGS

FIG. 1 is a schematic diagram showing the overall configuration of the system including the motion analysis device 10 according to an embodiment of the present invention.
FIG. 2 is a block diagram showing the software configuration of the motion analysis device 10.
FIG. 3 is a block diagram showing the hardware configuration of the computer 100 which constitutes the motion analysis apparatus 10.
FIG. 4 is a flowchart showing the procedures of motion analysis.
FIG. 5 is a diagram illustrating an example of joints and their connection relationships in body tracking.
FIG. 6 is a diagram showing an example of the method for defining the coordinate system used in coordinate transformation.
FIG. 7 is a diagram showing the position and trajectory of the right ankle joint while walking, measured by using Azure Kinect and VICON.
FIG. 8A is a diagram showing the positions of the center of the shoulder joint and center of the hip joint measured by using Azure Kinect and VICON.
FIG. 8B is a diagram showing the trajectory of the center of the shoulder joint while walking measured by using Azure Kinect and VICON.
FIG. 8C is a diagram showing the trajectory of the center of the hip joint center while walking measured by using Azure Kinect and VICON.

EMBODIMENT FOR CARRYING OUT THE INVENTION

In the following, by referring the drawings, the typical embodiments of the coordinate transformation method, coordinate transformation device, motion analysis method, motion analysis device, and computer programs according to the present invention are explained in detail. However, the present invention is not particularly limited to the drawings. Further, since these drawings are presented to explain the concept of the present invention, there are cases where sizes, ratios and numbers are exaggerated or simplified as necessary for ease of understanding.

1. With respect to motion analysis device 10 and coordinate transformation device

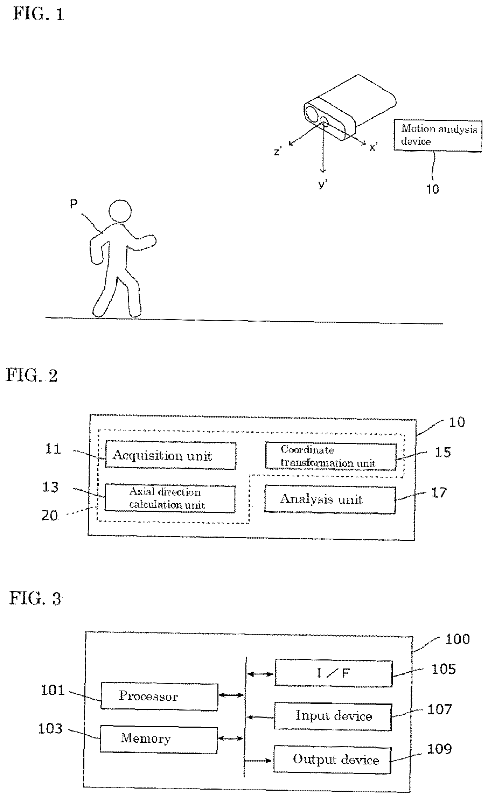

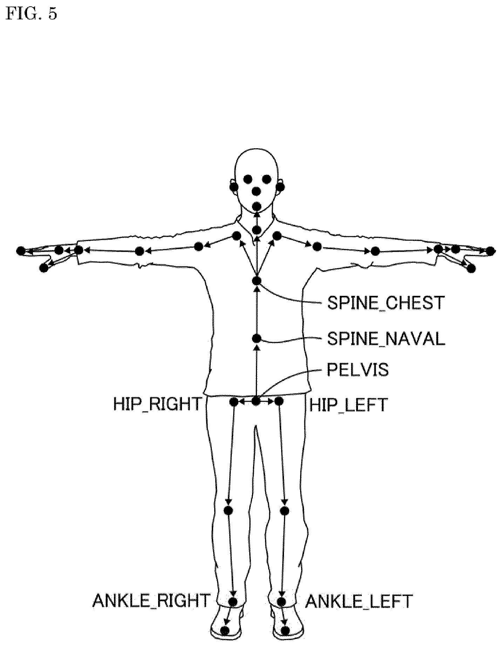

The motion analysis device 10 is a device or system that performs motion analysis by using the three-dimensional coordinate data obtained by photographing the subject P. Examples of the subject P include, patients undergoing rehabilitation, patients undergoing examinations, athletes, and the like, but not limited thereto. That is, the subject P is moving in some way, for example, by travelling such as walking or running, or by exercising such as jumping or gymnastics, and is in motion.
As shown in FIG. 2, the motion analysis device 10 includes an acquisition unit 11, an axial direction calculation unit 13, a coordinate transformation unit 15, and an analysis unit 17. Among these components, the acquisition unit 11, the axial direction calculation unit 13, and the coordinate transformation unit 15 constitute a coordinate transformation device 20.
The acquisition unit 11 acquires three-dimensional skeletal information of the subject P in motion. Here, the skeletal information includes three-dimensional coordinate data.
The acquisition unit 11 may be one that primitively acquires (i.e. generates) skeletal information, or may be one that secondarily acquires skeletal information by transfer or the like.
In the former case, the acquisition unit 11 can be configured as a body tracking system that acquires image data including depth information by using a photographing device such as an IR camera (depth sensor), generates a human skeletal model (skeleton) from the image data (see FIG. 5), and can measure the three-dimensional coordinate of joint point.
Alternatively, in the latter case, the acquisition unit 11 may be a communication interface for receiving the three-dimensional skeletal information from this type of the body tracking system, or a memory for storing the skeletal information.

An example of the body tracking system is Azure Kinect (trademark) from Microsoft Corporation, but is not limited thereto, and examples of the body tracking system may include Intel RealSense Depth (trademark) Camera from Intel Corporation, ASTRA (trademark) 3D camera from Orbbec 3D Tech. Intl. Inc., Xtion (trademark) from ASUSTeK Computer Inc., and ZED (trademark) camera from Stereolabs Inc.

The skeletal information acquired by the acquisition unit 11 is written in a coordinate system (hereinafter, sometimes referred to as an original coordinate system or a first coordinate system) fixed to the body tracking system. For example, in Azure Kinect, the origin of the three-dimensional coordinate values is the center of the IR camera (depth sensor), and the coordinate axes are set as the x' axis for the horizontal axis, the y' axis for the vertical axis, and the z' axis for the depth axis when viewed from the front of the IR camera (see FIG. 1).

The acquisition unit 11 acquires the skeletal information at predetermined time intervals. The predetermined time interval may be the same as the generation of image data or skeletal information by the body tracking system, and is, for example, 30 fps.

Next, the axial direction calculation unit 13 uses the acquired skeletal information to calculate a new coordinate system (hereinafter, sometimes referred to as a second coordinate system). That is, the axial directions of the new coordinate system are the Y direction, which is the direction of travelling of the subject P, the Z direction along the torso of the subject P, and the X direction perpendicular to the Y direction and the Z direction. The Z direction is preferably a direction from the pelvis of the subject P toward the chest. Note that, it is arbitrary which direction is named X, Y, and Z directions.

In this embodiment, the Y direction is set to the direction from the position of SPINE_NAVAL at the time before walking starts to the position of SPINE_NAVAL at the time after walking ends. That is, the Y direction can be obtained by subtracting the coordinate of SPINE_NAVAL at the time before walking starts from the coordinate of SPINE_NAVAL at the time after walking ends.

Further, the Z' direction is set to the direction from the PELVIS position to the SPINE_NAVAL position. That is, the Z' direction can be obtained by subtracting the coordinate of PELVIS from the coordinate of SPINE_NAVAL at the same time. As the Z' direction, an average of multiple Z' directions calculated over a predetermined period of time may be used.

The X direction is defined to be perpendicular to the Y direction and the Z' direction, and the Z direction is defined to be perpendicular to the X direction and the Y direction.

The calculated vector indicating the X direction, the vector indicating the Y direction, and the vector indicating the Z direction may be normalized to a unit length.

Next, the coordinate transformation unit 15 transforms the original coordinate system describing the skeleton information into a new coordinate system having the X direction, the Y direction, and the Z direction as coordinate axes. For example, the coordinate transformation unit 15 can perform transformation to the new coordinate system by using a matrix corresponding to the original coordinate system and a matrix corresponding to the new coordinate system.

Here, considering the case where the position p of a point in an original coordinate system whose axes are three mutually orthogonal unit vectors i', j', and k' is transformed into a new coordinate system whose axes are three other orthogonal unit vectors i, j, and k that share the origin with this original coordinate system.

If the coordinates of the position p in each coordinate system are (x', y', z') and (x, y, z), the position p can be expressed as follows.

[Equation 1]

$$\boldsymbol{p} = x\,\boldsymbol{i} + y\,\boldsymbol{j} + z\,\boldsymbol{k} = x'\,\boldsymbol{i}' + y'\,\boldsymbol{j}' + z'\,\boldsymbol{k}'$$

This equation can be written by using matrices as follows.

[Equation 2]

$$(\boldsymbol{i} \quad \boldsymbol{j} \quad \boldsymbol{k})\begin{pmatrix} x \\ y \\ z \end{pmatrix} = (\boldsymbol{i}' \quad \boldsymbol{j}' \quad \boldsymbol{k}')\begin{pmatrix} x' \\ y' \\ z' \end{pmatrix}$$

Therefore, the position p(x, y, z) in the new coordinate system is given by the following equation.

[Equation 3]

$$\begin{pmatrix} x \\ y \\ z \end{pmatrix} = (\boldsymbol{i} \quad \boldsymbol{j} \quad \boldsymbol{k})^{-1} (\boldsymbol{i'} \quad \boldsymbol{j'} \quad \boldsymbol{k'}) \begin{pmatrix} x' \\ y' \\ z' \end{pmatrix}$$

Here, since the matrices (i, j, k) and (i', j', k') are orthogonal matrices, their inverse matrices are equal to their transpose matrices.

[Equation 4]

$$\begin{pmatrix} x \\ y \\ z \end{pmatrix} = (\boldsymbol{i} \quad \boldsymbol{j} \quad \boldsymbol{k})^{T} (\boldsymbol{i'} \quad \boldsymbol{j'} \quad \boldsymbol{k'}) \begin{pmatrix} x' \\ y' \\ z' \end{pmatrix}$$

Therefore, a transformation matrix M is obtained, which transforms the position representation (x', y', z') in the original coordinate system into the position representation (x, y, z) in the new coordinate system.

[Equation 5]

$$M = (\boldsymbol{i} \quad \boldsymbol{j} \quad \boldsymbol{k})^{T} (\boldsymbol{i'} \quad \boldsymbol{j'} \quad \boldsymbol{k'}) = \begin{pmatrix} \boldsymbol{i} \cdot \boldsymbol{i'} & \boldsymbol{i} \cdot \boldsymbol{j'} & \boldsymbol{i} \cdot \boldsymbol{k'} \\ \boldsymbol{j} \cdot \boldsymbol{i'} & \boldsymbol{j} \cdot \boldsymbol{j'} & \boldsymbol{j} \cdot \boldsymbol{k'} \\ \boldsymbol{k} \cdot \boldsymbol{i'} & \boldsymbol{k} \cdot \boldsymbol{j'} & \boldsymbol{k} \cdot \boldsymbol{k'} \end{pmatrix}$$

Accordingly, by applying the transformation matrix M to the representation (x', y', z') in the original coordinate system from the left side, the representation (x, y, z) in the new coordinate system can be obtained.

The analysis unit 17 performs a motion analysis by using the coordinate-transformed skeletal information.

Examples of the motion analysis include gait analysis, running analysis, swimming analysis, jumping analysis, and gymnastics analysis, and the like, but other analyses of the above-mentioned movements (travelling through walking, running or swimming, or some movement through exercise such as jumping or gymnastics) are also possible, and, for example, analysis of movements such as running and standing long jump is suitable.

In particular, analysis of movements such as walking, running, swimming, and jumping includes, for example, walking cycle, walking speed, joint angle, posture, stride length, step width, foot height, arm (elbow) swing range, arm (elbow) height, left-right balance, and front-back and left-right sway of the body, and the like, but is not limited thereto.

The results of the motion analysis are displayed on a display, printed, or transmitted to another computer for use by medical doctors, physical therapists, researchers, and the like. Based on the results of the motion analysis, these persons can create treatment methods that are individually suited to the subject P and utilize them for purposes such as treatment and rehabilitation.

2. With respect to the computer 100 that constitutes the motion analysis device 10 and the coordinate transformation device 20

As shown in FIG. 3, the computer 100 includes a processor 101, a memory 103, and a communication interface 105, and may further include an input device 107 and an output device 109. The computer 100 may be configured as a single computer or may be configured as multiple computers.

The processor 101 reads out various programs and data into the memory 103 and executes them to realize various functions of the motion analysis device 10 and the coordinate transformation device 20. The processor 101 may be configured by a semiconductor integrated circuit such as a central processing unit (CPU), a graphics processing unit (GPU), or a microprocessor.

The memory 103 is a random access memory (RAM) and a read only memory (ROM) that store various data and programs. The memory 103 includes a non-transitory computer-readable storage medium such as a hard disk drive, a solid state drive, or a flash memory.

The communication interface 105 is an interface for connecting to wired and wireless communication networks, for example, an adapter for connecting to Ethernet (registered trademark), a modem for connecting to a public telephone line network, a wireless communication device for wireless communication, a USB (Universal Serial Bus) connector or an RS232C connector for serial communication, or the like.

The input device 107 is, for example, a keyboard, a mouse, a touch panel, a button, a microphone, or the like, for inputting various data. Further, the output device 109 is, for example, a display, a printer, a speaker, or the like, for outputting various data.

3. Motion analysis method and coordinate transformation method

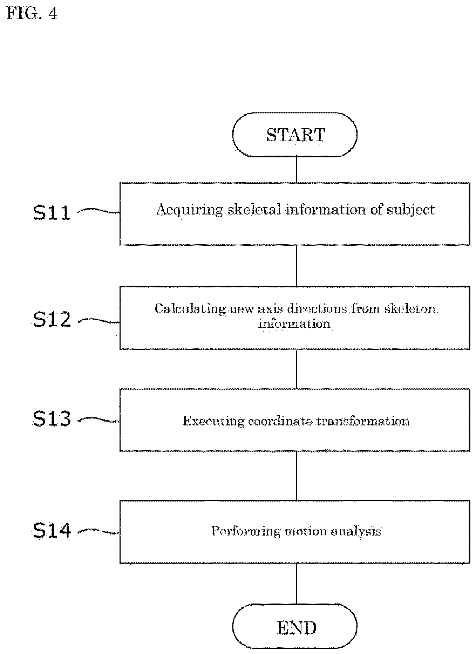

With reference to FIG. 4, the motion analysis method including the coordinate transformation method will be explained. The motion analysis method is a computer-implemented method. Among the procedures constituting the motion analysis method, steps S11 to S13 correspond to the coordinate transformation method.

In step S11, the skeletal information of the subject P is acquired. At this time, the skeletal information is described in the coordinate system (X', Y', Z' axes; unit vectors i', j', k') fixed to the body tracking system or camera (see FIG. 1). Further, the skeletal information is acquired at predetermined time intervals.

Next, in step S12, the X, Y, and Z axis directions (unit vectors i, j, k) of the new coordinate system are calculated from the skeleton information. For example, the direction of travelling is the Y direction, the direction along the torso of the subject P is the Z direction, and the direction perpendicular to the Y direction and the Z direction is the X direction.

Then, in step S13, coordinate transformation of the skeleton information is executed. For example, the above-mentioned transformation matrix M is generated and applied to the original coordinate system representation (x', y', z') from the left side to obtain the new coordinate system representation (x, y, z).

Furthermore, in step S14, the transformed coordinates are used to perform the motion analysis such as a gait analysis.

Then, the series of processes ends.

4. Effects of this embodiment

As is seen from this embodiment, the inventors have defined the new coordinate system for motion analysis based on the motion data such as walking measured by using the motion capture system such as Azure Kinect, and developed an algorithm for performing the coordinate transformation.

In the analysis of the walking movements by the conventional markerless motion capture systems, the above-mentioned problems have been solved by focusing on parameters that are independent of the coordinate system such as joint angle and angular velocity, or by previously acquiring information for performing coordinate transformation (such as markers installed in the laboratory). In this embodiment, information regarding the coordinate transformation required for motion analysis can be obtained from the measurement data, which makes it possible to perform the detailed analysis of the movements such as walking without using a complex system.

5. Analysis Example

Here, in case of the gait analysis as an example of the motion analysis, the results of verifying the accuracy of the motion analysis in this embodiment are shown.

In this case, in order to evaluate the accuracy of the gait analysis, a comparison was made with the data measured by using VICON (trademark) available from Vicon Motion Systems Ltd. VICON is a type of the motion capture system that uses an infrared camera to track retroreflective markers attached to the body of the subject P, and is thought to provide accurate and reliable data.

In this analysis example, Azure Kinect (trademark) is used as the motion capture system. Azure Kinect is a markerless motion capture system that can automatically fit a human skeletal model (skeleton) from a depth image obtained by projecting an infrared dot pattern onto a subject, and measure the coordinates of 20 joint points (see FIG. 5).

In Azure Kinect, the built-in processor estimates the joint portions of the body and reads the three-dimensional coordinate values (x, y, z) of the joint data when constructing skeletal information. The origin of the three-dimensional coordinate values read by the joint data is the center of the IR camera (depth sensor), and the coordinate axes, when viewed from the front of the IR camera, are X on the horizontal axis, y on the vertical axis, and Z on the depth axis (see FIG. 1). Note that, the IR camera measures the distance (depth) from the camera to an object by using infrared rays.

In Azure Kinect, the origin of the coordinates is at the focal point of the camera. The coordinate system is set up so that the positive X-axis points to the right, the positive Y-axis points down, and the positive Z-axis points forward. Therefore, in Azure Kinect, the joint positions are expressed as relative values with respect to the reference frame of the depth sensor. That is, the skeletal information of the subject P is represented in the coordinate system fixed to Azure Kinect.

Here, the subject P may travel in any desired direction relative to the camera of the motion capture system. In this case, in the above coordinate system, depending on the direction of travelling of the subject P, the measurement accuracy for joints hidden from the camera may deteriorate.

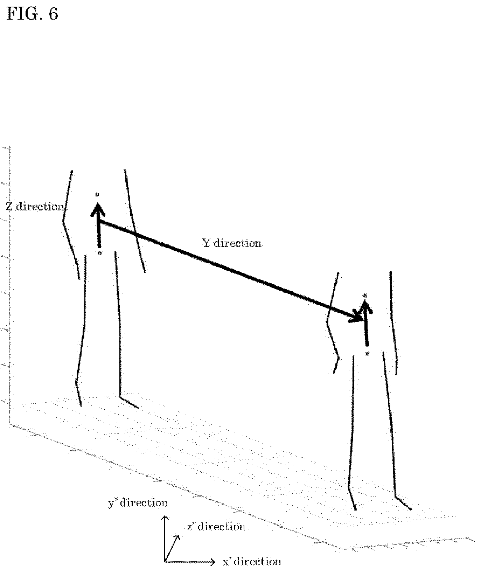

Further, the axes of the new coordinate system are set as follows (see FIG. 6).

Y direction: Direction from SPINE_NAVEL at the start of walking to SPINE_NAVEL at the end of walking

Z' direction: Direction from PELVIS to SPINE_CHEST while walking (average)
X direction: Direction perpendicular to the Y axis and Z' axis
Z direction: Direction perpendicular to the X axis and Y axis

5-1. Measuring the position of the ankle while walking

The positions of the ankles of subject P while walking were measured by using Azure Kinect and VICON, and the trajectories were compared. However, the measurement results by using Azure Kinect are subject to the coordinate transformation described above.

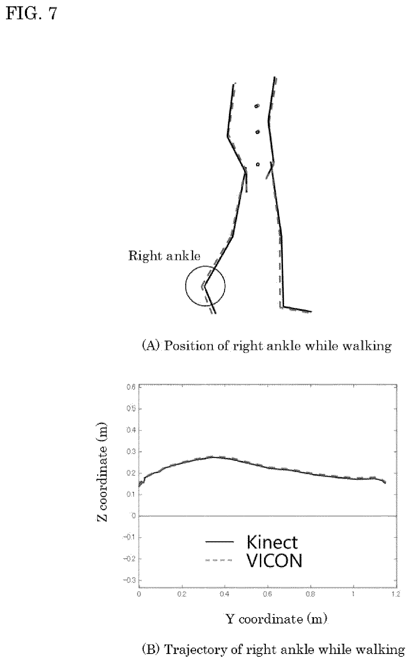

An example of the measurement results for the right ankle is shown in FIG. 7(A) and (B). The illustrated example is the measurement result when subject P walks diagonally forward relative to the camera, and the original coordinate system (x', y', z' axes) is displayed for reference.

Further, the correlation coefficient between the positions of the left and right ankles while walking measured by using Azure Kinect and VICON is shown in Table 1.

[Tabel 1]

|  | X (left-right) direction | Y (front-back) direction | Z (vertical) direction |
|---|---|---|---|
| Right ankle | .474 ± .444 | .953 ± .120 | .742 ± .430 |
| Left ankle | .460 ± .499 | .952 ± .125 | .745 ± .455 |

In general, with respect to the correlation coefficient r, a positive correlation is recognized when $0.4 \leqq r \leqq 0.7$, and a strong positive correlation is recognized when $0.7 \leqq r \leqq 1$. Therefore, from Table 1, it can be seen that there is a particularly strong correlation in the front-back direction and the vertical direction.

Further, the root mean square error of the positions of the left and right ankles while walking measured by using Azure Kinect and VICON is shown in Table 2.

[Table 2]

| (m) | X (left-right) | Y (front-back) | Z (vertical) | XYZ |
|---|---|---|---|---|
| Right ankle | 0.022 ± 0.014 | 0.107 ± 0.170 | 0.036 ± 0.029 | 0.123 ± 0.167 |
| Left ankle | 0.020 ± 0.014 | 0.099 ± 0.153 | 0.039 ± 0.044 | 0.114 ± 0.156 |

From Table 2, it can be seen that the errors are particularly small in the left-right direction and the vertical direction.

Further, the stride length (average) can be calculated from the trajectory of the ankle position. In the following, Table 3 shows an example of left and right stride lengths while walking measured by using Azure Kinect and VICON.

[Table 3]

| (m) | Kinect | VICON |
|---|---|---|
| Right | 1.17 ± 0.12 | 1.13 ± 0.18 |
| Left | 1.17 ± 0.12 | 1.16 ± 0.12 |

From Table 3, it can be seen that the difference in the measurement values between Azure Kinect and VICON is within a few percent.

5-2. Midpoints of left and right hip joints

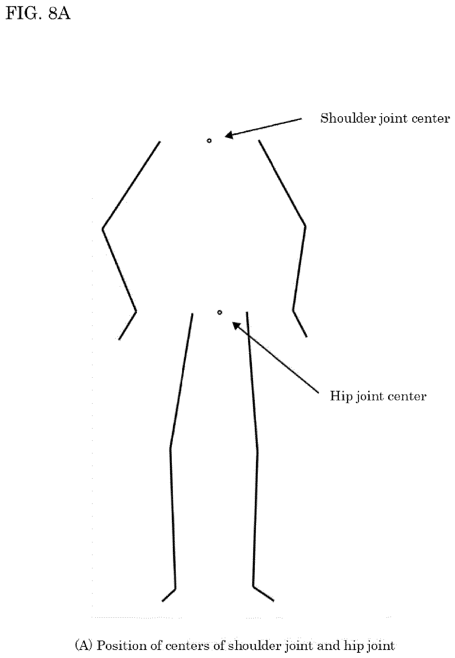

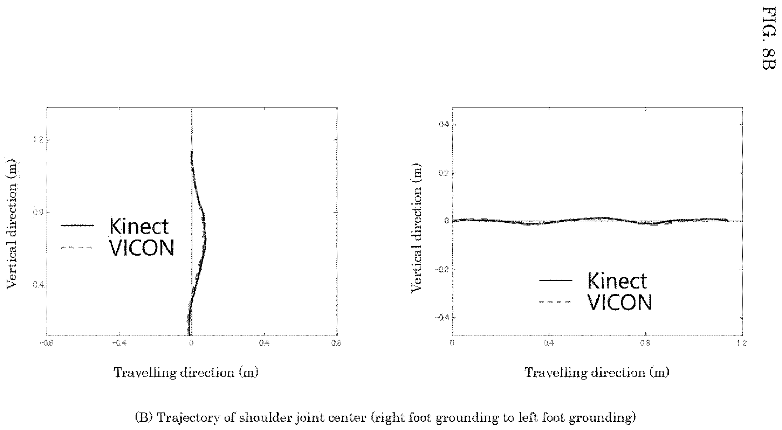

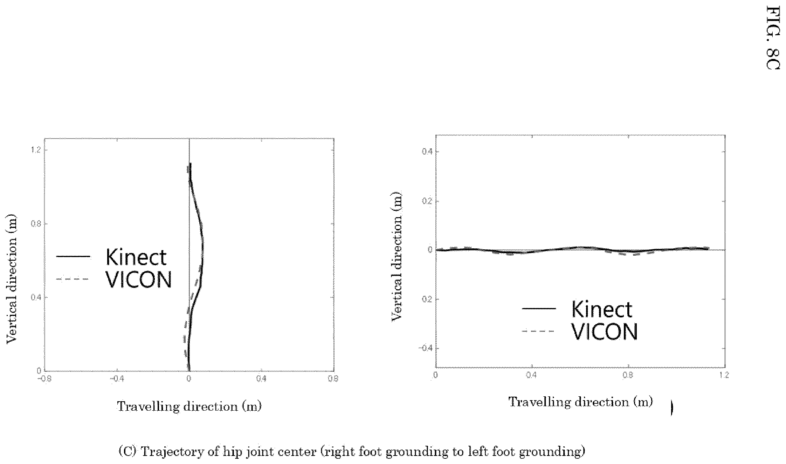

The positions of the centers of the left and right shoulder joints and the left and right hip joints of the subject P (see FIG. 8A) were measured by using Azure Kinect and VICON, and the trajectories were compared (see FIG. 8B and FIG. 8C). The measurement results by using Azure Kinect are subject to the coordinate transformation described above.

In the following, Table 4 shows the root mean square error of the measured left and right shoulder and hip centers.

[Table 4]

| (m) | X (left-right) | Y (front-back) | Z (vertical) | XYZ |
|---|---|---|---|---|
| Sholder joint center | 0.016 ± 0.014 | 0.030 ± 0.071 | 0.011 ± 0.005 | 0.046 ± 0.070 |
| Hip joint center | 0.021 ± 0.012 | 0.032 ± 0.075 | 0.012 ± 0.006 | 0.048 ± 0.071 |

Further, the correlation coefficients of the measured shoulder joint center and hip joint center are shown in Table 5.

[Table 5]

| | X (left-right) direction | Y (front-back) direction | Z (vertical) direction |
|---|---|---|---|
| Shoulder joint center | .741 ± .503 | .999 ± .006 | .610 ± .399 |
| Hip joint center | .665 ± .447 | .996 ± .005 | .660 ± .345 |

From Table 5, it can be seen that there is a particularly strong correlation in the left-right direction and the front-back direction.

These comparative examples confirmed that the coordinate-converted measurement data from Azure Kinect had accuracy comparable to that of the measurement data from VICON. Therefore, it is thought that the coordinate-transformed measurement results of Azure Kinect accurately represent the positions of the joints of the subject P.

Therefore, the coordinate transformation technique presented here can be suitably used for the motion analysis including gait analysis.

Accordingly, for reliable motion analysis, there is no need to install multiple cameras, nor is there a need to place markers on the floor, and the like, so by using coordinate transformation technology, it is possible to easily build the motion capture system.

In the above, although the typical embodiments of the present invention have been described, the present invention is not limited to these, and various design changes are possible, and all of those are included in the present invention.

EXPLANATION OF SYMBOLS

10 Motion analysis device
11 Acquisition unit
13 Axial direction calculation unit
15 Coordinate transformation unit
17 Analysis unit
20 Coordinate transformation device

**Claims**

1. A coordinate transformation method **characterized by** carrying out procedures which comprise:

   acquiring three-dimensional skeletal information of a subject in motion in a first coordinate system comprising a x' direction, a y' direction, and a z' direction, which are mutually orthogonal;
   using the skeletal information to calculate a Y direction being the traveling direction of the subject, a Z direction along the torso of the subject, and a X direction orthogonal to the Y direction and the Z direction; and
   transforming the first coordinate system into a second coordinate system comprising the X direction, the Y direction, and the Z direction, and

   thereby acquiring coordinate-transformed skeletal information.

2. The coordinate transformation method according to claim 1, wherein the Z direction is a direction from a pelvis to a chest of the subject.

3. The coordinate transformation method according to claim 1, wherein by using a matrix corresponding to the first coordinate system and a matrix corresponding to the second coordinate system, the first coordinate system is transformed into the second coordinate system.

4. A computer program for carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction; and
transforming the first coordinate system into a second coordinate system comprising the X direction, the Y direction, and the Z direction, and

thereby acquiring coordinate-transformed skeletal information.

5. A coordinate transformation device comprising:

an acquisition unit that acquires three-dimensional skeletal information of a subject in motion in a first coordinate system comprising a x' direction, a y' direction, and a z' direction, which are mutually orthogonal;
an axial direction calculation unit that, by using the skeletal information, calculates a Y direction being the traveling direction of the subject, a Z direction along the torso of the subject, and a X direction orthogonal to the Y direction and the Z direction; and
a coordinate transformation unit that transforms the first coordinate system into a second coordinate system comprising the X direction, the Y direction, and the Z direction.

6. A motion analysis method **characterized by** carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction;
transforming the first coordinate system into a second coordinate system comprising the X direction, the Y direction, and the Z direction; and
performing motion analysis using the coordinate-transformed skeletal information.

7. A computer program for carrying out procedures which comprise:

acquiring three-dimensional skeletal information of a subject in motion in a first coordinate system comprising a x' direction, a y' direction, and a z' direction, which are mutually orthogonal;
using the skeletal information to calculate a Y direction being the traveling direction of the subject, a Z direction along the torso of the subject, and a X direction orthogonal to the Y direction and the Z direction;
transforming the first coordinate system into a second coordinate system comprising the X direction, the Y direction, and the Z direction; and
performing motion analysis using the coordinate-transformed skeletal information.

8. A motion analysis device comprising:

an acquisition unit that acquires three-dimensional skeletal information of a subject in motion in a first coordinate system comprising a x' direction, a y' direction, and a z' direction, which are mutually orthogonal;
an axial direction calculation unit that, by using the skeletal information, calculates a Y direction being the traveling direction of the subject, a Z direction along the torso of the subject, and a X direction orthogonal to the Y direction and the Z direction;
a coordinate transformation unit that transforms the first coordinate system into a second coordinate system comprising the X direction, the Y direction, and the Z direction; and
an analysis unit that performs motion analysis using the coordinate-transformed skeletal information.

FIG. 1

Motion analysis device

10

P

FIG. 2

10

11 — Acquisition unit

15 — Coordinate transformation unit

13 — Axial direction calculation unit

20

17 — Analysis unit

FIG. 3

100

101 — Processor

103 — Memory

105 — I／F

107 — Input device

109 — Output device

FIG. 4

```
                    ┌─────────────┐
                    │    START    │
                    └──────┬──────┘
                           │
S11  ──┐   ┌───────────────────────────────────────┐
       └───│  Acquiring skeletal information of subject │
           └───────────────────┬───────────────────┘
                               │
S12  ──┐   ┌───────────────────────────────────────┐
       └───│  Calculating new axis directions from skeleton │
           │              information               │
           └───────────────────┬───────────────────┘
                               │
S13  ──┐   ┌───────────────────────────────────────┐
       └───│     Executing coordinate transformation     │
           └───────────────────┬───────────────────┘
                               │
S14  ──┐   ┌───────────────────────────────────────┐
       └───│        Performing motion analysis         │
           └───────────────────┬───────────────────┘
                               │
                    ┌──────────────────┐
                    │       END        │
                    └──────────────────┘
```

FIG. 5

SPINE_CHEST

SPINE_NAVAL

PELVIS

HIP_RIGHT          HIP_LEFT

ANKLE_RIGHT          ANKLE_LEFT

FIG. 6

FIG. 7

(A) Position of right ankle while walking

Y coordinate (m)

(B) Trajectory of right ankle while walking

FIG. 8A

Shoulder joint center

Hip joint center

(A) Position of centers of shoulder joint and hip joint

FIG. 8B

(B) Trajectory of shoulder joint center (right foot grounding to left foot grounding)

FIG. 8C

(C) Trajectory of hip joint center (right foot grounding to left foot grounding)

## INTERNATIONAL SEARCH REPORT

| International application No. |
| :--- |
| **PCT/JP2024/004768** |

**A. CLASSIFICATION OF SUBJECT MATTER**

*A61B 5/11*(2006.01)i; *G06T 7/00*(2017.01)i; *G06T 7/20*(2017.01)i; *G06V 40/20*(2022.01)i
FI:   A61B5/11 230; G06T7/00 660B; G06T7/20 300Z; G06V40/20

According to International Patent Classification (IPC) or to both national classification and IPC

**B. FIELDS SEARCHED**

Minimum documentation searched (classification system followed by classification symbols)

A61B5/11; G06T7/00; G06T7/20; G06V40/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| :---: | :--- | :---: |
| X | JP 2022-65241 A (HITACHI HIGH-TECH CORPORATION) 27 April 2022 (2022-04-27) paragraphs [0019], [0037]-[0039], fig. 7 | 1-8 |
| X | JP 2016-80671 A (MIZUSAWA, Junichi) 16 May 2016 (2016-05-16) paragraphs [0026]-[0040], [0053]-[0058], [0067] | 1-8 |
| A | CN 115471867 A (GUANGZHOU VIRTUAL POWER NETWORK TECHNOLOGY CO., LTD.) 13 December 2022 (2022-12-13) entire text, all drawings | 1-8 |
| A | CN 111035393 A (SHENZHEN INSTITUTES OF ADVANCED TECHNOLOGY, CHINESE ACADEMY OF SCIENCES) 21 April 2020 (2020-04-21) entire text, all drawings | 1-8 |
| A | CN 108830215 A (NANJING UNIVERSITY OF SCIENCE AND TECHNOLOGY) 16 November 2018 (2018-11-16) entire text, all drawings | 1-8 |

☐ Further documents are listed in the continuation of Box C.     ☑ See patent family annex.

| | |
| :--- | :--- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| :--- | :--- |
| **15 April 2024** | **07 May 2024** |

| Name and mailing address of the ISA/JP | Authorized officer |
| :--- | :--- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/004768**

| Patent document cited in search report | | | Publication date (day/month/year) | Patent family member(s) | Publication date (day/month/year) |
|---|---|---|---|---|---|
| JP | 2022-65241 | A | 27 April 2022 | US 2023/0377375 A1 paragraphs [0036], [0055]-[0057], fig. 7 WO 2022/080165 A1 | |
| JP | 2016-80671 | A | 16 May 2016 | (Family: none) | |
| CN | 115471867 | A | 13 December 2022 | (Family: none) | |
| CN | 111035393 | A | 21 April 2020 | (Family: none) | |
| CN | 108830215 | A | 16 November 2018 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6381918 B